# EUROPÄISCHE PATENTSCHRIFT

(11) **EP 0 787 477 B1**
(45) Veröffentlichungstag und Bekanntmachung des Hinweises auf die Patenterteilung: **21.08.2002**
(21) Anmeldenummer: 97100985.7
(22) Anmeldetag: 23.01.1997
(51) Int. Cl.: A61K 6/083, A61K 6/09

(54) **Adhäsive**
Adhesives
Adhésifs

(30) Priorität: 01.02.1996 DE 19603577
(43) Veröffentlichungstag der Anmeldung: 06.08.1997
(73) Patentinhaber: Heraeus Kulzer GmbH & Co.KG, 63450 Hanau (DE)
(72) Erfinder: Finger, Werner, Prof. Dr., 41469 Neuss (DE); Podszun, Wolfgang, Dr., 51061 Köln (DE)
(74) Vertreter: Kühn, Hans-Christian

(56) Entgegenhaltungen:
- EP-A- 0 113 926
- EP-A- 0 282 280
- EP-A- 0 393 617
- US-A- 5 362 769

## Beschreibung

Die Erfindung betrifft eine Zubereitung zur Verwendung als Adhäsivkomponente, vorzugsweise für die Behandlung der Zahnhartsubstanz und von Dentallegierungen.

Ein besonders gravierendes Problem in der konservierenden Zahnheilkunde besteht in der dauerhaften, randspaltfreien Verklebung von Kunststoff-Füllungsmaterialien mit der Zahnhartsubstanz (Dentin und Schmelz). Im Dentalbereich werden aushärtbare Materialien als Füllungsmaterialien bei Zahnreparaturen verwendet. Als aushärtbare Materialien werden im allgemeinen Füllungsmaterialien auf Acrylatbasis bevorzugt, die durch radikalische Polymerisation in der Zahnkavität ausgehärtet werden. Ein Nachteil dieser Materialien besteht darin, daß sie während der Aushärtung schrumpfen und so zur Bildung von Randspalten zwischen der Kavitätenwandung und der Zahnfüllung beitragen. Die Kunststoff-Füllungen haben den zusätzlichen Nachteil, daß sie schlecht am Dentin haften bleiben.

Um die Bindung an die Zahnhartsubstanz zu verbessern, kann man sogenannte Dentaladhäsive einsetzen. Dabei werden Dentaladhäsive bevorzugt, die sowohl am Dentin als auch am Schmelz gute Bindungsfestigkeiten ergeben. Wirksame Formulierungen enthalten im allgemeinen eine Vielzahl von Komponenten. So wird in DE-A-38 28 170 ein Beschichtungsmittel für kollagenhaltige Materialien beschrieben, das aus
a) Aldehyd,
b) einem wasserlöslichen Monomer mit aktivem Wasserstoff,
c) einem wasserunlöslichen Monomer mit zwei oder mehreren polymerisierbaren Doppelbindungen,
d) einem Photoinitiator,
e) Wasser,
f) einem Löslichkeitsvermittler und/oder Dispergator und
g) bekannten Zusätzen
besteht. Mit diesem Beschichtungsmittel lassen sich Bindungsfestigkeiten von 11,8 - 19 N/mm² an Dentin und von 12,6 - 17 N/mm² an Schmelz erreichen.

In DE-A-41 05 550 wird eine Zubereitung aus
a) einem Formamidoalkylmethacrylat,
b) einem (Meth)Acrylsäureester, der Vernetzungen ausbilden kann,
c) einem Lösungsmittel,
d) gegebenenfalls weiteren Zusätzen,
e) einer Säure und
f) gegebenenfalls einem Dispergator
als Dentaladhäsiv beschrieben. Mit dieser Zubereitung lassen sich Bindungsfestigkeiten von 7,2 - 9,9 N/mm² an Dentin und von 9,6 - 14,4 N/mm² an Schmelz erreichen.

Es wurde nun eine Zubereitung gefunden, die wenige Komponenten enthält, sehr einfach angewendet werden kann und sehr hohe Bindungsfestigkeiten an Schmelz, Dentin, Keramik, Metallen und Metall-Legierungen ermöglicht.

Die erfindungsgemäße Zubereitung ist dadurch gekennzeichnet, daß sie aus
a) 10 bis 40 Gew.-% Hydroxyalkyl(meth)acrylat,
b) 10 bis 40 Gew.-% Urethandi(meth)acrylat,
c) 30 bis 70 Gew.-% eines flüchtigen, mit Wasser mischbaren Lösungsmittels,
d) 0,01 bis 2,5 Gew.-% Photoinitiator und
e) 0 bis 40 Gew.-% an sich bekannter Zusätze
besteht.

Hydroxyalkyl(meth)acrylate im Rahmen der Erfindung sind Hydroxy-Gruppen aufweisende Ester von mehrwertigen Alkoholen, vorzugsweise von Glykolen, mit Acrylsäure oder Methacrylsäure. Beispielhaft seien genannt: 2-Hydroxyethylacrylat, 2-Hydroxyethylmethacrylat, 2-Hydroxypropylacrylat, 2-Hydroxypropylmethacrylat und 2,3-Dihydroxypropylmethacrylat (Glycerinmonomethacrylat). Bevorzugt werden 2-Hydroxyethylmethacrylat und 2,3-Dihydroxypropylmethacrylat (Glycerinmonomethacrylat).

Die erfindungsgemäßen Urethandi(meth)acrylate sind Umsetzungsprodukte von Diisocyanaten mit Hydroxyalkyl(meth)acrylaten. Die Urethandi(meth)acrylate können sich von aliphatischen, verzweigt-aliphatischen, cyclo-aliphatischen oder aromatischen Diisocyanaten ableiten. Bevorzugt werden die Umsetzungsprodukte von aliphatischen, verzweigt-aliphatischen und cyclo-aliphatischen Diisocyanaten. Beispielhaft seien die folgenden Urethandiacrylate (UDA) und Urethandimethacrylate (UDM) genannt:

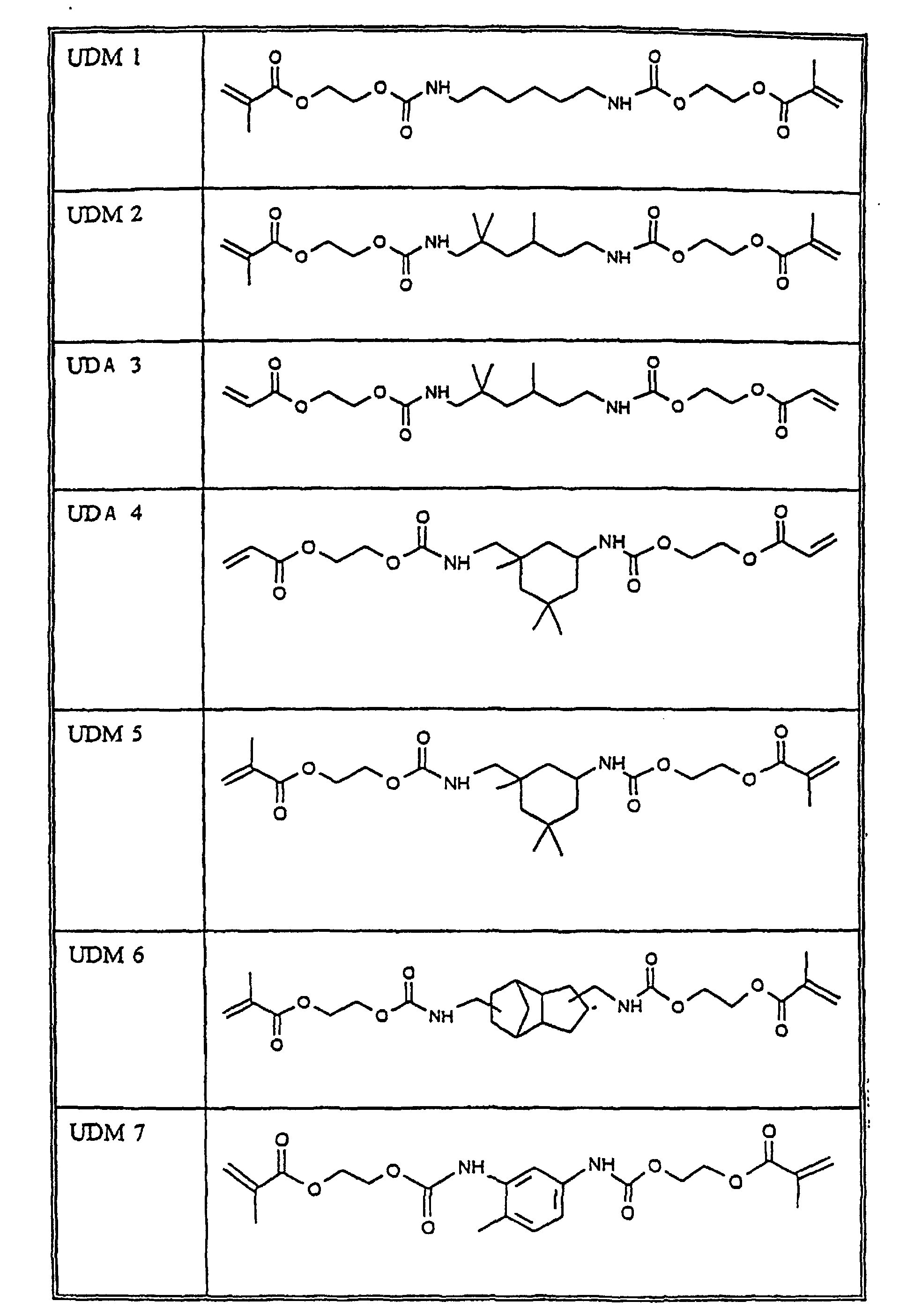

Flüchtige, mit Wasser mischbare Lösungsmittel sind vor allem solche mit einem Dampfdruck von mindestens 100 Torr bei Raumtemperatur. Bevorzugt sind aliphatische Alkohole mit ein bis vier C-Atomen, Aceton, 1,4-Dioxan und Tetrahydrofuran. Besonders bevorzugt sind Aceton und Ethylalkohol.

Photoinitiatoren im Rahmen der vorliegenden Erfindung sind Radikalbildner, die unter der Einwirkung von Licht, beispielsweise UV-Licht, sichtbarem Licht oder Laserlicht, eine radikalische Polymerisation auslösen.

Diese Photopolymerisationsinitiatoren sind an sich aus der Literatur bekannt. Vorzugsweise handelt es sich um Mono- oder Dicarbonylverbindungen, wie Benzophenon, Benzoin und dessen Derivate, insbesondere Benzoinmethylether, Benzil und Benzilderivate und andere Dicarbonylverbindungen, wie Diacetyl, 2,3-Pentandion und α-Diketoderivate des Norbomans und substituierter Norbornane, Metallcarbonyle, wie Pentacarbonylmangan, oder Chinone, wie 9,10-Phenanthrenchinon und Naphthochinon. Besonders bevorzugt ist Campherchinon.

Die erfindungsgemäße Zubereitung enthält im allgemeinen 0,01 bis 2,5 Gew.-%, bevorzugt 0,1 bis 0,5 Gew.-%, des Photoinitiators, bezogen auf die erfindungsgemäße Zubereitung.

Es kann vorteilhaft sein, der erfindungsgemäßen Zubereitung Coaktivatoren zuzusetzen, die die Polymerisationsreaktion beschleunigen. Bekannte Beschleuniger sind beispielsweise Amine, wie p-Toluidin, Dimethyl-p-toluidin, Trialkylamine, wie Trihexylamin, Polyamine, wie N,N,N',N'-Tetraalkylalkylendiamine, Barbitursäure und Dialkylbarbitursäuren. Besonders bevorzugt sind Dimethylaminobenzolsulfonamide entsprechend DE-A-31 35 113.

Die Coaktivatoren werden im allgemeinen in einer Menge von 0,02 bis 4 Gew.-%, bevorzugt 0,2 bis 1 Gew.-%, bezogen auf die erfindungsgemäße Zubereitung, eingesetzt.

Die erfindungsgemäße Zubereitung kann neben Hydroxy(meth)acrylat, Urethandi(meth)acrylat, Lösungsmittel, Photoinitiator und Coaktivator gegebenenfalls weitere (Meth)Acrylsäureester als Comonomere enthalten. Bevorzugt seien Ester der (Meth)Acrylsäure mit 1- bis 5-wertigen Alkoholen mit 2 bis 30 Kohlenstoffatomen genannt.

Außerdem seien Derivate des Tricyclodecans (EP-A-0 023 686) und Umsetzungsprodukte aus Polyolen, Diisocyanaten und Hydroxyalkylmethacrylaten (DE-A-37 03 120, DE-A-37 03 080 und DE-A-37 03 130) genannt.

Besonders bevorzugt als (Meth)acrylsäureester wird das sogenannte Bis-GMA der Formel

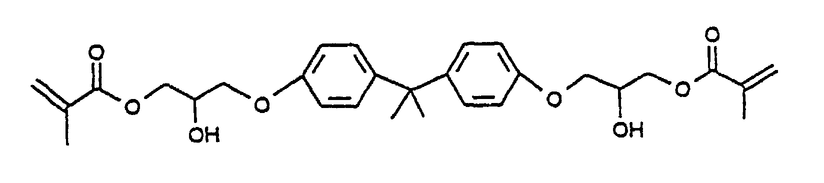

Die erfindungsgemäße Zubereitung ermöglicht nicht nur eine gute Bindung von Kunststoff an Dentin und Schmelz, sondern auch an Keramik, Metallen und Metall-Legierungen.

In einer besonderen Ausführungsform der Erfindung enthält die erfindungsgemäße Zubereitung Methacryloyloxy-Gruppen aufweisende Ester aromatischer Tri- oder Tetracarbonsäuren zur weiteren Verbesserung der Bindung von Kunststoff an Metallen, vorzugsweise an Dentallegierungen. Anstelle der Tri- oder Tetracarbonsäuren können auch die entsprechenden Derivate eingesetzt werden, bei denen zwei benachbarte Carbonsäuregruppen eine Anhydridgruppe bilden. Als geeignete Methacryloyloxy-Gruppen aufweisende Ester aromatischer Tri- oder Tetracarbonsäuren seien die folgenden Verbindungen beispielhaft aufgeführt:

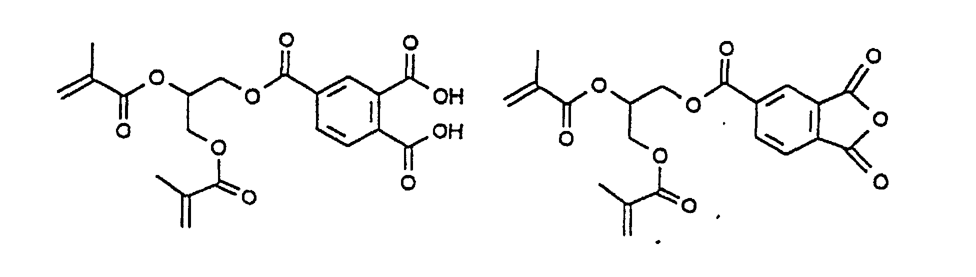

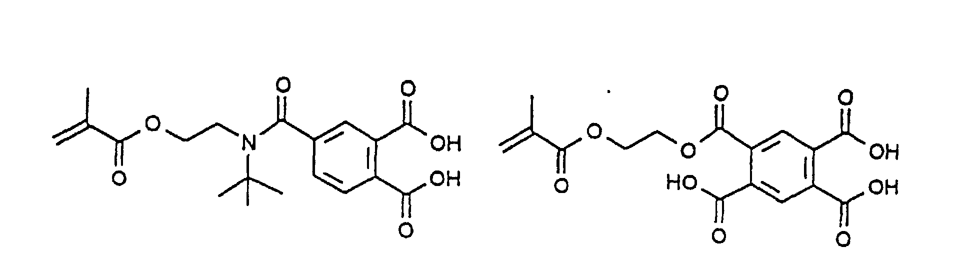

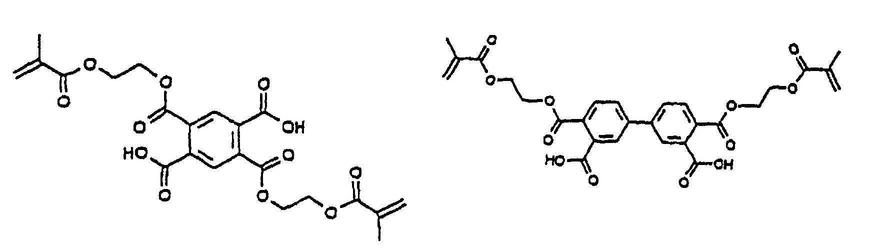

Besonders gut geeignet sind die in der Fachliteratur als "4-MET" und "4-META" bezeichneten Derivate der Trimellitsäure der Formeln

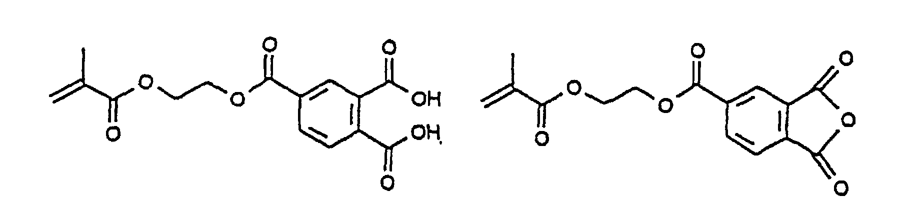

Die Methacryloyloxy-Gruppen aufweisenden Ester aromatischer Tricarbonsäuren werden in Mengen von 2 bis 30 Gew.-%, vorzugsweise 5 bis 20 Gew.-%, bezogen auf die Zubereitung, angewendet.

Eine besonders gute Elastizität der durch Polymerisation ausgehärteten Schicht der Zubereitung wird erzielt, wenn die Zubereitung zusätzlich Polyethylenglykoldi(meth)acrylate, insbesondere Polyethylenglykoldi(meth)acrylate mit einem Molekulargewicht von 200 - 2000, enthält. Andere Zusätze zur Verbesserung der elastischen Eigenschaften - sogenannte Elastifizierungsmittel - können aus Polyester(meth)acrylaten, Polyesterpolyurethan(meth)acrylaten oder aus anderen Polyether(meth)acrylaten bestehen. Die Elastifizierungsmittel werden in Mengen von 1 bis 30 Gew.-%, vorzugsweise 5 bis 20 Gew.-%, bezogen auf die Zubereitung, angewendet.

Es wurde gefunden, daß die an sich sehr hohen Bindungsfestigkeiten an Dentin durch einen Anteil an Füllstoff noch weiter gesteigert werden können. Geeignete Füllstoffe sind beispielsweise Bergkristall, Kristobalit, Quarzglas, hochdisperse Kieselsäuren, Aluminiumoxid und Glaskeramiken. Die mittlere Teilchengröße der Füllstoffe liegt im allgemeinen im Bereich von 5 bis 2000 nm, vorzugsweise im Bereich von 10 - 100 nm. Besonders gut geeignete Füllstoffe sind hochdisperse Kieselsäuren, die beispielsweise durch Flammhydrolyse gewonnen werden können. Besonders wirksam sind Zusätze in einer Menge von 5 bis 20 Gew.-%, bezogen auf die Zubereitung.

Die Füllstoffe werden vorzugsweise vorbehandelt, beispielsweise mit Silanisierungsmitteln aus Organosilicium-Verbindungen (Progress in Organic Coatings 11, 297 - 308 (1983). Ein bevorzugtes Silanisierungsmittel ist 3-Methacryloyloxypropyl-trimethoxysilan.

Die Zubereitung gemäß der Erfindung kann weiterhin übliche Zusätze, wie Stabilisatoren, Inhibitoren und Lichtschutzmittel, enthalten.

Die erfindungsgemäße Zubereitung kann in einfacher Weise durch Mischen der einzelnen Komponenten hergestellt werden.

In einer bevorzugten Ausführungsform der Erfindung werden ein Diisocyanat und ein Überschuß an Hydroxyalkyl(meth)acrylat in einem geeigneten Lösungsmittel, wie z. B. Aceton, in Anwesenheit eines Katalysators zusammengegeben, wobei eine Mischung aus Hydroxyalkyl(meth)acrylat, Urethandi(meth)acrylat und Lösungsmittel erhalten wird. Als Katalysator können Metallsalze höherer Fettsäuren, wie z. B. Dibutylzinndilaurat, Triarylverbindungen, wie z. B. Triphenylstibin oder Triphenylphosphin, oder tertiäre Amine, wie z. B. Triethylamin, eingesetzt werden. Durch Zugabe eines Photoinitiators und gegebenenfalls weiterer Zusätze zu der Mischung wird eine erfindungsgemäße Zubereitung erhalten.

Die erfindungsgemäße Zubereitung eignet sich als Adhäsivkomponente zur Behandlung von Keramik, Metallen und Metall-Legierungen und wird vorzugsweise zur Behandlung der Zahnhartsubstanz und von Dentallegierungen verwendet

In einer besonderen Ausführungsform konditioniert man die Zahnhartsubstanz vor der Behandlung mit der erfindungsgemäßen Zubereitung mit einer Konditionierungsflüssigkeit, die einen pH-Wert im Bereich von 0,1 bis 3,5 aufweist. Die Konditionierungsflüssigkeit enthält im aligemeinen Säuren mit einem pKₛ-Wert kleiner als 5 und gegebenenfalls eine amphotere Aminoverbindung mit einem pKₛ-Wert im Bereich von 9,0 bis 10,6 und einem pK_{b}-Wert im Bereich von 11,5 bis 12,5. Folgende Säuren können z. B. in der Konditionierungsflüssigkeit enthalten sein: Phosphorsäure, Salpetersäure, Brenztraubensäure, Zitronensäure, Oxalsäure, Ethylendiamintetraessigsäure, Essigsäure, Weinsäure und Äpfelsäure. Weiterhin kann die Konditionierungsflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden, solange freie Säurefunktionen verbleiben. Bevorzugt wird die Behandlung mit wäßriger Phosphorsäure. Geeignete Konzentrationen der Phosphorsäure sind 10 bis 60 Gew.-%, vorzugsweise 20 bis 40 Gew.-%. Die Konditionierungsflüssigkeit kann zur Einstellung einer geeigneten Konsistenz Verdickungsmittel, wie z. B. Kieselsäure, enthalten.

Die Anwendung der erfindungsgemäßen Zubereitung kann beispielsweise wie folgt durchgeführt werden:

Bei einer Zahnreparatur trägt man nach einer mechanischen Reinigung der Zahnoberfläche zuerst die Konditionierungsflüssigkeit auf, läßt eine kurze Zeit (beispielsweise 60 Sekunden) einwirken, spült die Zahnoberfläche mit Wasser und trocknet sie. Dann trägt man die erfindungsgemäße Zubereitung, beispielsweise mit einem kleinen Pinsel, in einer oder mehreren dünnen Schichten auf, trocknet im Luftstrom und bestrahlt mit einer handelsüblichen Polymerisationslampe. Danach trägt man das eigentliche Füllungsmaterial, beispielsweise ein im Dentalbereich übliches Kunststoff-Füllungsmaterial, auf.

Zur näheren Erläuterung werden im Folgenden Beispiele für die erfindungsgemäße Zubereitung (Beispiele 1 - 7, 12 und 15), die Herstellung einer erfindungsgemäßen Zubereitung in einer bevorzugten Ausführungsform (Beispiel 7, simultane Herstellung), die Herstellung eines Polyesterpolyurethanmethacrylats (Beispiel 11) und die Prüfung der Wirksamkeit der Zubereitung a) durch Bestimmung der Scherbindungsfestigkeit von Kunststoff-Füllungen auf Dentin und gegebenenfalls Schmelz (Beispiele 8, 13 und 16) und auf Dentallegierungen (Beispiel 10) und b) durch Bestimmung der Kavitätenadaptation (Beispiele 9 und 14) nach Vorbehandlung mit der Zubereitung beschrieben. Das in den Beispielen benutzte Siliciumdioxid besitzt eine mittlere Teilchengröße von 14 Nanometer. Die Beispiele 1 A und 5 A sind nicht erfindungsgemäß.

### Beispiel 1 A - F

Die folgenden Zubereitungen werden durch intensives Vermischen der Bestandteile erzeugt.

| Beispiel 1 | A | B | C | D | E | F |
|---|---|---|---|---|---|---|
| Urethandimethacrylat UDM 2 | 5,0 g | 4,0 g | 3,0 g | 2,5 g | 2,0 g | 1,0 g |
| 2-Hydroxyethylmethacrylat | - | 1,0 g | 2,0 g | 2,5 g | 3,0 g | 4,0 g |
| Aceton | 5,0 g | 5,0 g | 5,0 g | 5,0 g | 5,0 g | 5,0 g |
| Campherchinon | 20 mg | 20 mg | 20 mg | 20 mg | 20 mg | 20 mg |
| Diallylsulfonamid∗ | 50 mg | 50 mg | 50 mg | 50 mg | 50 mg | 50 mg |

### Beispiel 2 A - E

Die folgenden Zubereitungen werden durch intensives Vermischen der Bestandteile erzeugt.

| Beispiel 2 | A | B | C | D | E |
|---|---|---|---|---|---|
| Urethandimethacrylat UDM 2 | 4,0 g | 3,0 g | 2,5 g | 2,0 g | 1,0 g |
| Glycerinmonomethacrylat | 1,0 g | 2,0 g | 2,5 g | 3,0 g | 4,0 g |
| Aceton | 5,0 g | 5,0 g | 5,0 g | 5,0 g | 5,0 g |
| Campherchinon | 20 mg | 20 mg | 20 mg | 20 mg | 20 mg |
| Diallylsulfonamid∗ | 50 mg | 50 mg | 50 mg | 50 mg | 50 mg |

### Beispiel 3 A - D

Die folgenden Zubereitungen werden durch intensives Vermischen der Bestandteile erzeugt.

| Beispiel 3 | A | B | C | D |
|---|---|---|---|---|
| Urethandi(meth)acrylat | 2,5 g UDM 1 | 2,5 g UDA 4 | 2,5 g UDM 6 | 2,5 g UDM 7 |
| 2-Hydroxyethylmethacrylat | 2,5 g | 2,5 g | 2,5 g | 2,5 g |
| Aceton | 5,0 g | 5,0 g | 5,0 g | 5,0 g |
| Campherchinon | 20 mg | 20 mg | 20 mg | 20 mg |
| Diallylsulfonamid∗ | 50 mg | 50 mg | 50 mg | 50 mg |

### Beispiel 4 A - D

Die folgenden Zubereitungen werden durch intensives Vermischen der Bestandteile erzeugt.

| Beispiel 4 | A | B | C | D |
|---|---|---|---|---|
| Urethandimethacrylat UDM 2 | 2,5 g | 2,5 g | 2,5 g | 2,5 g |
| 2-Hydroxyethylmethacrylat | 2,5 g | 2,5 g | 2,5 g | 2,5 g |
| Aceton | 5,0 g | 5,0 g | 5,0 g | 5,0 g |
| silanisiertes Siliciumdioxid ∗∗ | 0,5 g | 1,0 g | 2,0 g | 3,0 g |
| Campherchinon | 20 mg | 20 mg | 20 mg | 20 mg |
| Diallylsulfonamid∗ | 50 mg | 50 mg | 50 mg | 50 mg |

### Beispiel 5 A - F

Die folgenden Zubereitungen werden durch intensives Vermischen der Bestandteile erzeugt.

| Beispiel 5 | A | B | C | D | E | F |
|---|---|---|---|---|---|---|
| Urethandimethacrylat UDM 2 | 5,0 g | 4,0 g | 3,0 g | 2,5 g | 2,0 g | 1,0 g |
| 2-Hydroxyethylmethacrylat | - | 1,0 g | 2,0 g | 2,5 g | 3,0 g | 4,0 g |
| silanisiertes Siliciumdioxid∗∗ | 1,0 g | 1,0 g | 1,0 g | 1,0 g | 1,0 g | 1,0 g |
| Aceton | 5,0 g | 5,0 g | 5,0 g | 5,0 g | 5,0 g | 5,0 g |
| Campherchinon | 20 mg | 20 mg | 20 mg | 20 mg | 20 mg | 20 mg |
| Diallylsulfonamid∗ | 50 mg | 50 mg | 50 mg | 50 mg | 50 mg | 50 mg |

### Beispiel 6 A - D

Die folgenden Zubereitungen werden durch intensives Vermischen der Bestandteile erzeugt.

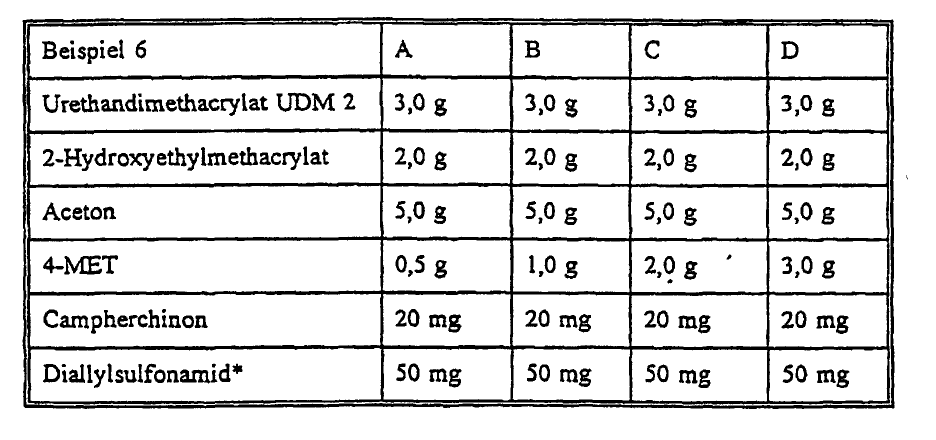

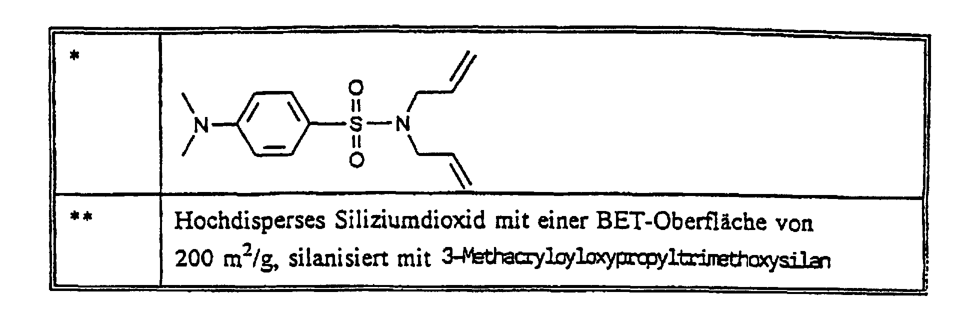

### Beispiel 7

### Simultane Herstellung von UDM 1 und erfindungsgemäßer Zubereitung

Ein Gemisch aus 1 g Hexamethylendiisocyanat, 4 g 2-Hydroxyethylmethacrylat, 5 g Aceton und 4 mg Dibutylzinndilaurat (Katalysator) wird 10 Stunden bei 40° C gerührt. Nach dem Abkühlen werden 1 g mit 3-Methacryloyloxypropyl-trimethoxysilan silanisiertes Siliciumdioxid, 20 mg Campherchinon und 50 mg Diallylsulfonamid zugesetzt.

### Beispiel 8

### Bestimmung der Scherbindungsfestigkeit an Dentin

Die Wirksamkeit der in den Beispielen 1 - 7 beschriebenen Zubereitungen wird geprüft durch Bestimmung der Scherbindungsfestigkeit auf Dentin. Es werden menschliche Zähne benutzt, die für max. drei Monate nach der Extraktion in 1 % Chloraminlösung aufbewahrt worden waren. Vor der Verwendung im Bindungstest werden die Zähne nach sorgfältiger Reinigung unter fließendem Wasser für mindestens drei und höchstens zehn Tage in physiologischer Kochsalzlösung gelagert. Am Tage vor der Verwendung im Bindungstest werden die Zähne einzeln auf einer Approximalseite liegend mit Expoxidharz (Lekutherm® X20, Härter T3) in zylindrische Gummiformen von 25 mm Durchmesser und 12 mm Höhe eingebettet. Die Zähne werden durch Naßschleifen mit SiC-Papieren der Körnungen 240, 320, 400 und 600 soweit beschliffen, daß eine ausreichend große, schmelznahe Dentinfläche zur Anbindung eines Kunststoffzylinders mit 3,5 mm Durchmesser freiliegt. Nach Abspülen mit entionisiertem Wasser und Trocknung im Luftstrom wird 30 Sekunden lang das Konditionierungsmittel Gluma® CPS Gel (20 % H₃PO₄, Bayer AG) mit einem Wattepellet unter reibender Bewegung aufgetragen, mit Wasser sorgfältig abgespült und durch Abtupfen mit Zellstoff oberflächlich von Wasser befreit (feuchte Technik). Auf die konditionierte Dentinfläche werden die Zubereitungen aus den Beispielen 1 - 7 mit einem Pinsel in drei Schichten aufgetragen, im Druckluftstrom getrocknet und mit dem Lichtgerät TRANSLUX® CL (Kulzer) 20 Sekunden lang bestrahlt. Die so vorbehandelten Proben werden dann mittels einer Einspannvorrichtung unter zweigeteilten zylindrischen Teflonformen (3,5 mm Durchmesser, 1 mm Höhe) festgeklemmt. Danach wird das Kunststoff-Füllungsmaterial PEKAFILL® (U, Bayer AG) mit einer Spritze in die Teflonformen gefüllt, mit einem O₂-undurchlässigen Strip abgedeckt und mit dem Lichtgerät TRANSLUX® CL 60 Sekunden lang bestrahlt. Unmittelbar anschließend werden die Teflonformen abgenommen und die Proben für 24 Stunden in 37° C warmem Wasser gelagert bis zur Einleitung der Scherbelastung. Dazu werden die mit Kunststoffzylindem versehenen Proben mit Hilfe eines Druckstempels parallel zu und dicht an der angeschliffenen Zahnoberfläche bei einer Geschwindigkeit von 1 mm/Minute bis zur Trennung des Zylinders vom Zahn belastet. Die Scherbindungsfestigkeit ist der Quotient aus Bruchkraft und Bindungsareal und wird jeweils an 5 Proben bestimmt und als deren Mittelwert in der Tabelle I angegeben.

### Bestimmung der Scherbindungsfestigkeit an Schmelz

Für die Bestimmung der Scherbindungsfestigkeit an mit den in den Beispielen 1-7 beschriebenen Zubereitungen behandeltem Schmelz werden extrahierte menschliche Zähne mit intakter labialer Schmelzfläche in Epoxidharz eingebettet und mit nassem SiC-Papier der Körnung 240 bis 600 angeschliffen, um eine plane periphere Schmelzoberfläche freizulegen. Auf die Schmelzoberfläche wird das Konditionierungsmittel Gluma® CPS Gel aufgetragen, das nach 30 Sekunden sorgfältig mit entionisiertem Wasser abgespült wird. Die Trocknung erfolgt nur oberflächlich mit einem schwachen Druckluftstrahl, bis die behandelte Oberfläche kreidig weiß erscheint. Alle weiteren Arbeitsschritte sind identisch mit den vorstehend beschriebenen zur Bestimmung der Scherbindungsfestigkeit an Dentin. Die Werte für die Scherbindungsfestigkeit an Schmelz werden in der Tabelle I angegeben.

**Tabelle I**

| Zubereitung | Scherbindungsfestigkeit an Dentin | Scherbindungsfestigkeit an Schmelz |
|---|---|---|
| Beispiel 1A | 8,2 MPa | 33,7 MPa |
| Beispiel IB | 17,7 MPa | 31,0 MPa |
| Beispiel 1C | 17,2 MPa | 35,1 MPa |
| Beispiel 1D | 17,2 MPa | 33,0 MPa |
| Beispiel 1E | 18,3 MPa | 33,8 MPa |
| Beispiel 1F | 16,5 MPa | 28,3 MPa |
| Beispiel 2A | 11,3 MPa | nicht bestimmt |
| Beispiel 2B | 12,7 MPa | nicht bestimmt |
| Beispiel 2C | 14,7 MPa | nicht bestimmt |
| Beispiel 2D | 11,4 MPa | nicht bestimmt |
| Beispiel 2E | 13,2 MPa | nicht bestimmt |
| Beispiel 3A | 17,9 MPa | nicht bestimmt |
| Beispiel 3B | 19,7 MPa | nicht bestimmt |
| Beispiel 3C | 17,6 MPa | nicht bestimmt |
| Beispiel 3D | 17,5 MPa | nicht bestimmt |
| Beispiel 4A | 15,3 MPa | 33,4 MPa |
| Beispiel 4B | 18,8 MPa | 34,8 MPa |
| Beispiel 4C | 18,1 MPa | 26,6 MPa |
| Beispiel 4D | 17,1 MPa | 31,1 MPa |
| Beispiel 5A | 12,7 MPa | 29,6 MPa |
| Beispiel 5B | 19,8 MPa | 32,1 MPa |
| Beispiel 5C | 18,7 MPa | 33,5 Mpa |
| Beispiel 5D | 18,0 MPa | 32,4 MPa |
| Beispiel 5E | 17,6 MPa | 35,4 MPa |
| Beispiel 5F | 19,0 MPa | 29,7 MPa |
| Beispiel 6A | 21,0 MPa | nicht bestimmt |
| Beispiel 6B | 25,0 MPa | nicht bestimmt |
| Beispiel 6C | 20,4 MPa | nicht bestimmt |
| Beispiel 6D | 16,5 MPa | nicht bestimmt |
| Beispiel 7 | 19,3 MPa | 32,3 MPa |

### Beispiel 9

### Kavitätenadaptation

Eine weitere Aussage über die Wirksamkeit eines Dentaladhäsivs läßt sich aufgrund von Untersuchungen des Randes von Zahnfüllungen machen, die in mit dem Dentaladhäsiv vorbehandelte Zahnkavitäten gelegt wurden. In der Zahnheilkunde werden in der Regel intradentale Defekte (von Zahnhartsubstanz umgebene Kavitäten) mit Füllungsmaterialien gefüllt. Dabei entstehen besonders bei Kunststoff-Füllungsmaterialien aufgrund der bei der Härtung auftretenden Schrumpfung Wand-zu-Wand-Kontraktionsspannungen, die zur Ablösung der Kunststoff-Füllung von der Kavitätenwandung und damit zur Bildung von Randspalten im Bereich des Füllungsrandes führen können. Das Verhalten der Zahnfüllungen an der Kavitätenwandung wird als Kavitätenadaptation bezeichnet. Dabei bedeuten nicht grundsätzlich Materialien mit hoher Scherbindungsfestigkeit gute und Materialien mit geringer Scherbindungsfestigkeit schlechte Kavitätenadaptation.

Zur Prüfung der Kavitätenadaptation werden extrahierte, menschliche Molaren (Mahlzähne) verwendet, die für maximal 3 Monate nach Extraktion in 1 % Chloraminlösung gelagert worden waren. Die Zähne werden auf einer intakten Approximalseite mit nassen SiC-Papieren der Kömungen 240, 320, 400 und 600 bis zur Freilegung ausreichend großer, schmelznaher Dentinflächen plangeschliffen. Ausgehend von den plangeschliffenen Dentinflächen werden in das Dentin der Zähne unter Wasserkühlung mit feinkörnigen diamantierten Präparationsinstrumenten auf einem mit einem Mikromotor betriebenen zahnärztlichen Handstück zylinderförmige Kavitäten mit ca. 3 mm Durchmesser und einer Tiefe von 1,5 mm präpariert. Der Kantenwinkel der Kavitäten beträgt 90°. Die Vorbehandlung der Kavitäten erfolgt mit dem Konditionierungsmittel Gluma® CPS Gel für 30 Sekunden. Anschließend wird das Gel mit Wasser sorgfältig abgespült und jede Kavität mit einem Wattepellet oberflächlich getrocknet. Wie bei den Bindungstests werden dann in den Beispielen beschriebene Zubereitungen mit einem Pinsel in zwei bis drei Schichten auf die Kavitätenwandungen aufgetragen. Die nach dem Entfernen des Lösungsmittels mit Druckluft verbliebenen dünnen Schichten werden 20 Sekunden lang mit Licht bestrahlt (Translux® CL), bevor die Kavitäten mit dem Kunststoff-Füllungsmaterial Pekafill® (U, Bayer AG) gefüllt, mit einem lichtdurchlässigen Strip abgedeckt und 60 Sekunden lang mit Licht (Translux® CL) bestrahlt werden. Die mit den Füllungen versehenen Zähne werden sofort für 15 Minuten in entionisiertes Wasser (23° C) gelegt. Anschließend wird mit nassem SiC-Papier (Körnungen 600 und 4000) der Überschuß der Füllungen entfernt, bis der Rand der Kavitäten freiliegt. Unmittelbar danach erfolgt im Auflichtmikroskop (500-fache Vergrößerung) eine Inspektion des Randes. Sofern sich zwischen den Füllungen und den Kavitätenwandungen Randspalten gebildet haben, wird die maximale Breite der Randspalten mit Hilfe eines Okular-Schraubenmikrometers bestimmt. In der Tabelle II werden die Breite der Randspalten (Mittelwerte von je 6 Proben) und die Anzahl der Zahnfüllungen ohne Randspalt zwischen Kavitätenwandung und Füllung von je 6 Proben angegeben.

**Tabelle II**

| Zubereitung | Randspalt [µm] (n= 6) | Anzahl spaltfreier Füllungen |
|---|---|---|
| Beispiel 1A | 4,1 | 0 |
| Beispiel 1B | 1,4 | 1 |
| Beispiel 1C | 0,7 | 3 |
| Beispiel 1D | 0,7 | 2 |
| Beispiel 1E | 1,2 | 1 |
| Beispiel 1F | 2,3 | 0 |
| Beispiel 5A | 4,2 | 0 |
| Beispiel 5B | 1,8 | 0 |
| Beispiel 5C | 0,2 | 5 |
| Beispiel 5D | 0,3 | 5 |
| Beispiel 5E | 0,1 | 5 |
| Beispiel 5F | 0,8 | 1 |

### Beispiel 10

### Bestimmung der Scherbindungsfestigkeit an der Dentallegierung Levochrom

Zur Prüfung der Bindungsfestigkeit von Kunststoff-Füllungsmaterialien an mit der erfindungsgemäßen Zubereitung vorbehandelten Dentallegierungen wird die CrCo-Gußlegierung Levochrom (Bayer AG) eingesetzt. Aus der Legierung werden würfelförmige Proben gegossen und wie die extrahierten Zähne in Epoxidharz eingebettet. Die Proben werden mit SiC-Papier (Körnung 240 bis 600) angeschliffen, mit 50 µm Edelkorund abgestrahlt und 5 Minuten in entionisiertem Wasser im Ultraschallbad gereinigt. Nach dem Trocknen der Proben mit Druckluft werden einige der in den Beispielen beschriebenen Zubereitungen in zwei Schichten aufgetragen. Anschließend - nach Abdampfen des Lösungsmittels - werden die so vorbehandelten Proben 20 Sekunden lang mit dem Lichtgerät TRANSLUX® CL bestrahlt und entsprechend den Bindungstests an Dentin und Schmelz durch Photopolymerisation mit einem Zylinder (3,5 mm ⌀, 1,5 mm hoch) aus dem Kunststoff-Füllungsmaterial verbunden. Die Bestimmung der in der Tabelle III angegebenen Scherbindungsfestigkeiten erfolgt nach 24-stündiger Lagerung der Proben in 37° C warmem Wasser wie in Beispiel 8 beschrieben.

**Tabelle III**

| Zubereitung | Scherbindungsfestigkeit an Levochrom |
|---|---|
| Beispiel 1C | 16,6 MPa |
| Beispiel 6A | 19,4 MPa |
| Beispiel 6B | 20,9 MPa |
| Beispiel 6C | 20,8 MPa |
| Beispiel 6D | 23,3 MPa |

### Beispiel 11

### Herstellung eines Polyesterpolyurethanmethacrylates (PEUDMA)

In einem 250 ml-Dreihalskolben werden 41,26 g Polyesterdesmophen HN 200 mit einem mittleren Molekulargewicht von 2000 (aliphatischer, OH-terminierter Polyester aus Adipinsäure, Neopentylglycol und Hexandiol, Hersteller: Bayer AG), 0,06 g 2,6-Di-tert-butylkresol, 0,06 g Dibutylzinndilaurat und 120 ml absolutiertes Aceton vorgelegt. Bei Raumtemperatur werden unter Rühren 4,2 g Hexamethylendiisocyanat zugetropft. Das Reaktionsgemisch wird auf 55 °C erhitzt und solange gerührt, bis der NCO-Gehalt konstant bleibt (ca. 20 Stunden). Die Kontrolle des NCO-Gehalte erfolgt IR-spektroskopisch. Nach Zugabe von 1,3 g 2-Hydroxyethylmethacrylat wird noch weitere 16 Stunden bei 55 °C gerührt. Nach dieser Zeit ist kein NCO mehr nachweisbar, und die Reaktion ist beendet. Durch Zugabe von weiterem Aceton wird eine 25%ige Lösung eingestellt. Die Lösung ist gut fließfähig und kann direkt zur Herstellung von Zubereitungen gemäß den Beispielen 12 und 15 eingesetzt werden.

### Beispiel 12

Die folgenden Zubereitungen werden durch intensives Vermischen der Bestandteile erzeugt.

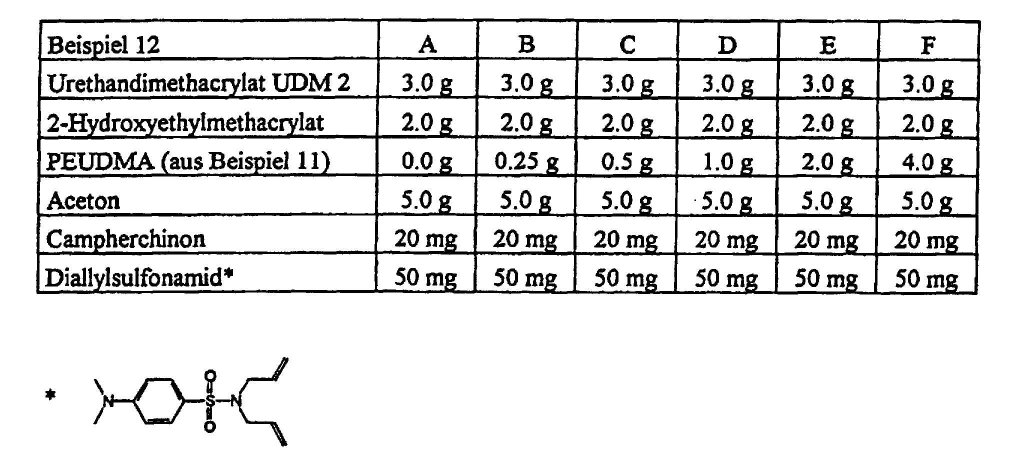

### Beispiel 13

### Bestimmung der Scherbindungsfestigkeit an Dentin

Die Zubereitungen aus Beispiel 12 werden entsprechend dem im Beispiel 8 beschriebenen Verfahren untersucht. Die Werte für die Scherbindungsfestigkeiten sind in Tabelle IV angegeben.

**Tabelle IV**

| Zubereitung | Scherbindungsfestigkeit an Dentin [MPa] |
|---|---|
| Beispiel 12A | 15.4 ± 1.2 |
| Beispiel 12B | 14.7 ± 1.1 |
| Beispiel 12C | 14.9 ± 1.0 |
| Beispiel 12D | 15.7 ± 1.4 |
| Beispiel 12E | 15.0 ± 1.4 |
| Beispiel 12F | 15.9 ± 2.9 |

### Beispiel 14

### Kavitätenadaptation

Die Zubereitungen aus Beispiel 12 werden entsprechend dem im Beispiel 9 beschriebenen Verfahren untersucht. Die Werte für Randspalt und Anzahl randspaltfreier Füllungen sind in Tabelle V angegeben.

**Tabelle V**

| Zubereitung | Randspalt [µm] (n=6) | Anzahl spaltfreier Füllungen |
|---|---|---|
| Beispiel 12A | 1.52 ± 1.31 | 2 |
| Beispiel 12B | 0 | 6 |
| Beispiel 12C | 0.48 ± 0.54 | 3 |
| Beispiel 12D | 1.12 ± 1.09 | 2 |
| Beispiel 12E | 1.13 ± 0.71 | 1 |
| Beispiel 12F | 3.47 ± 1.41 | 0 |

### Beispiel 15

Die folgenden Zubereitungen werden durch intensives Vermischen der Bestandteile erzeugt.

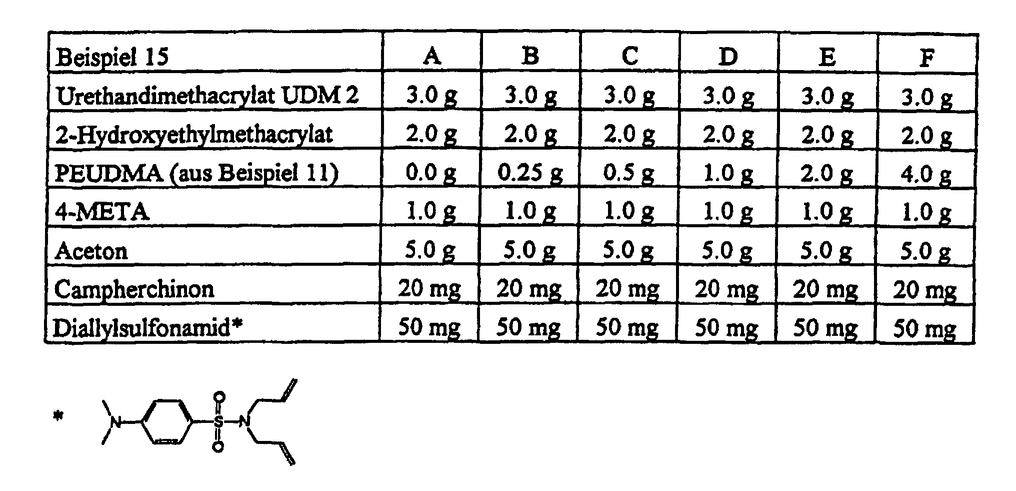

### Beispiel 16

### Bestimmung der Scherbindungsfestigkeit an Dentin

Die Zubereitungen aus Beispiel 15 werden entsprechend dem im Beispiel 8 beschriebenen Verfahren untersucht. Die Werte für die Scherbindungsfestigkeiten sind in Tabelle VI angegeben.

**Tabelle VI**

| Zubereitung | Scherbindungsfestigkeit an Dentin [MPa] |
|---|---|
| Beispiel 15A | 17.5 ± 1.8 |
| Beispiel 15B | 19.5 ± 4.7 |
| Beispiel 15C | 19.0 ± 3.5 |
| Beispiel 15D | 18.7 ± 1.2 |
| Beispiel 15E | 16.0 ± 2.5 |
| Beispiel 15F | 16.0 ± 3.6 |

## Patentansprüche

1. Zubereitung, **dadurch gekennzeichnet, daß** sie aus
a) 10 bis 40 Gew.-% Hydroxyalkyl(meth)acrylat,
b) 10 bis 40 Gew.-% Urethandi(meth)acrylat,
c) 30 bis 70 Gew.-% eines flüchtigen, mit Wasser mischbaren Lösungsmittels,
d) 0,01 bis 2,5 Gew.-% Photoinitiator und
e) 0 bis 40 Gew.-% an sich bekannter Zusätze besteht.

2. Zubereitung nach Anspruch 1, **dadurch gekennzeichnet, daß** sie e1) 5 bis 20 Gew.-% Füllstoff enthält.

3. Zubereitung nach Anspruch 2, **dadurch gekennzeichnet, daß** sie als Füllstoff hochdisperse Kieselsäure mit einer mittleren Teilchengröße von 5 bis 2000 nm enthält.

4. Zubereitung nach Anspruch 3, **dadurch gekennzeichnet, daß** sie als Füllstoff hochdisperse Kieselsäure mit einer mittleren Teilchengröße von 10 bis 100 nm enthält.

5. Zubereitung nach einem der Ansprüche 1 bis 4, **dadurch gekennzeichnet, daß** sie e2) 5 bis 30 Gew.-% Methacryloyloxygruppen aufweisende Ester aromatischer Tri- oder Tetracarbonsäuren oder der entsprechenden Anhydride enthält.

6. Zubereitung nach Anspruch 5, **dadurch gekennzeichnet, daß** sie e2) 5 bis 30 Gew.-% 4-MET oder 4-META enthält.

7. Verwendung der Zubereitung nach einem der Ansprüche 1 bis 6 zur Behandlung der Zahnhartsubstanz.

8. Verfahren zur Herstellung der Zubereitung nach einem der Ansprüche 1 bis 6, **dadurch gekennzeichnet, daß** Diisocyanat mit einem Überschuß an Hydroxyalkyl(meth)acrylat in einem flüchtigen, mit Wasser mischbaren Lösungsmittel in Anwesenheit eines Katalysators umgesetzt wird und nach erfolgter Umsetzung zum Urethandi(meth)acrylat die weiteren Komponenten zugesetzt werden.

9. Zubereitung nach einem der Ansprüche 1 bis 6, **dadurch gekennzeichnet, daß** sie e3) 1 bis 30 Gew.-% Polyether(meth)acrylate, Polyester(meth)acrylate oder Polyesterpolyurethan(meth)acrylate enthält.

10. Zubereitung nach Anspruch 9, **dadurch gekennzeichnet, daß** sie e3) 1 bis 30 Gew.-% Polyethylenglykoldi(meth)acrylat enthält.

## Claims

1. Formulation, **characterized in that** it consists of
a) 10 to 40% by weight of hydroxyalkyl (meth)acrylate,
b) 10 to 40% by weight of urethane di(meth)acrylate,
c) 30 to 70% by weight of a volatile, water-miscible solvent,
d) 0.01 to 2.5% by weight of a photoinitiator and
e) 0 to 40% by weight of additives known per se.

2. Formulation according to Claim 1, **characterized in that** it contains e1) 5 to 20% by weight of a filler.

3. Formulation according to Claim 2, **characterized in that** it contains, as a filler, colloidal silica having a mean particle size of 5 to 2000 nm.

4. Formulation according to Claim 3, **characterized in that** it contains, as a filler, colloidal silica having a mean particle size of 10 to 100 nm.

5. Formulation according to any of Claims 1 to 4, **characterized in that** it contains e2) 5 to 30% by weight of esters of aromatic tri- or tetracarboxylic acids, which esters have methacryloyloxy groups, or of the corresponding anhydrides.

6. Formulation according to Claim 5, **characterized in that** it contains e2) 5 to 30% by weight of 4-MET or 4-META.

7. Use of the formulation according to any of Claims 1 to 6 for the treatment of the hard tooth substance.

8. Process for the preparation of the formulation according to any of Claims 1 to 6, **characterized in that**. diisocyanate is reacted with an excess of hydroxyalkyl (meth)acrylate in a volatile, water-miscible solvent in the presence of a catalyst and the further components are added after the conversion into the urethane di(meth)acrylate is complete.

9. Formulation according to any of Claims 1 to 6, **characterized in that** it contains e3) 1 to 30% by weight of polyether (meth)acrylates, polyester (meth)acrylates or polyesterpolyurethane (meth)-acrylates.

10. Formulation according to Claim 9, **characterized in that** it contains e3) 1 to 30% by weight of polyethylene glycol di(meth)acrylate.

## Revendications

1. Préparation, **caractérisée en ce qu'**elle est constituée
a) de 10 à 40 % en poids de (méth)acrylate d'hydroxyalkyle,
b) de 10 à 40 % en poids de di(méth)acrylate d'uréthane,
c) de 30 à 70 % en poids d'un solvant volatil, miscible à l'eau,
d) de 0,01 à 2,5 % en poids d'un photo-initiateur et
e) de 0 à 40 % en poids d'additifs connus.

2. Préparation selon la revendication 1, **caractérisée en ce qu'**elle contient e1) de 5 à 20 % en poids de charge.

3. Préparation selon la revendication 2, **caractérisée en ce qu'**elle contient comme charge de l'acide silicique hautement dispersé avec une taille moyenne de particules de 5 à 2000 nm.

4. Préparation selon la revendication 3, **caractérisée en ce qu'**elle contient comme charge de l'acide silicique hautement dispersé avec une taille moyenne de particules de 10 à 100 nm.

5. Préparation selon l'une quelconque des revendications 1 à 4, **caractérisée en ce qu'**elle contient e2) de 5 à 30 % en poids d'ester présentant des groupes méthacryloyloxy d'acides tri- ou tétracarboxyliques aromatiques ou des anhydrides correspondants.

6. Préparation selon la revendication 5, **caractérisée en ce qu'**elle contient e2) de 5 à 30 % en poids de 4-MET ou de 4-META.

7. Utilisation de la préparation selon l'une quelconque des revendications 1 à 6 pour le traitement de la substance dure des dents.

8. Procédé pour préparer la préparation selon l'une quelconque des revendications 1 à 6, **caractérisé en ce que** l'on fait réagir un diisocyanate avec un excès de (méth)acrylate d'hydroxyalkyle dans un solvant volatil, miscible à l'eau en présence d'un catalyseur et **en ce que** l'on ajoute, après avoir réalisé la réaction au di(méth)acrylate d'uréthane les autres constituants.

9. Préparation selon l'une quelconque des revendications 1 à 6, **caractérisée en ce qu'**elle contient e3) de 1 à 30 % en poids de (méth)acrylate de polyéther, de (méth)acrylate de polyester ou de (méth)acrylate de polyesterpolyuréthane.

10. Préparation selon la revendication 9, **caractérisée en ce qu'**elle contient e3) de 1 à 30 % en poids de di(méth)acrylate de polyéthylèneglycol.
